# EUROPEAN PATENT SPECIFICATION

(11) **EP 2 221 274 B1**
(45) Date of publication and mention of the grant of the patent: **17.01.2018**
(21) Application number: 08847426.7
(22) Date of filing: 05.11.2008
(51) Int. Cl.: C23C 16/26, C23C 16/56, C01B 32/05

(54) **CARBONACEOUS THIN FILM AND MANUFACTURING METHOD FOR SAME**
KOHLENSTOFFHALTIGER DÜNNFILM UND HERSTELLUNGSVERFAHREN DAFÜR
FILM MINCE CARBONÉ ET SON PROCÉDÉ DE FABRICATION

(30) Priority: 07.11.2007 JP 2007289295
(43) Date of publication of application: 25.08.2010
(73) Proprietor: Toyo Advanced Technologies Co., Ltd., Hiroshima, 734-8501 (JP)
(72) Inventor: NITTA, Yuki, Hiroshima-shi Hiroshima 734-8501 (JP); NAKATANI, Tatsuyuki, Hiroshima-shi Hiroshima 734-8501 (JP); OKAMOTO, Keishi, Hiroshima-shi Hiroshima 734-8501 (JP)
(74) Representative: Zinnecker, Armin
(86) International application number: PCT/JP2008/003194
(87) International publication number: WO 2009/060602

(56) References cited:
- JP-A- 2006 213 569
- JP-T- 2001 501 299
- US-A1- 2007 207 321
- ZHANG, G.-J. ET AL.: 'DNA micropaterning on polycrystaline diamond via one-step direct amination' LANGMUIR vol. 22, no. 8, 11 April 2006, pages 3728 - 3734, XP008135999
- OKAMURA H. ET AL.: 'Rinsho Shindan'yo Kiban Gene Silicon no Kaihatsu', vol. 34, April 2004, TOYO KOHAN CO., LTD., ISSN 0372-3496 pages 41 - 46, XP008142308
- SZUNERITZ, S. ET AL.: 'Direct amination of hydrogen-terminated boron doped diamond surfaces' ELECTROCHEM. COMMUN. vol. 8, no. 7, July 2006, pages 1185 - 1190, XP025182234
- HARTL, A. ET AL.: 'Protein-modified nanocrystalline diamond thin films for biosensor applications' NATURE MAT. vol. 3, no. 10, October 2004, pages 736 - 742, XP008135966
- TAKAHASHI K. ET AL.: 'DNA Chip eno Diamond DLC no Tekiyo' NEW DIAMOND vol. 19, no. 4, 25 October 2003, ISSN 1340-4792 pages 7 - 12

## Description

### TECHNICAL FIELD

The present invention relates to carbon thin films and methods of forming the films, and more particularly to carbon thin films introduced with amino groups on the surfaces and methods of forming the films.

### BACKGROUND ART

Studies have been made to use carbon thin films represented by diamond like carbon (DLC) thin films in various fields, since the films are inactive and excellent in durability. In particular, application of the films to usage requiring biocompatibility and durability is expected, since the films have little interaction with biocomponents such as cells. For example, by coating a surface of a medical instrument such as a stent used in a living organism with a carbon thin film, improvements in antithrombogenicity and durability are expected.

On the other hand, as a method of further improving the biocompatibility of a carbon thin film, there is a technique for introducing a hydrophilic functional group into a carbon thin film. For example, the present inventors disclose generating a radical on a surface of a carbon thin film by irradiating the carbon thin film with plasma to perform graft polymerization using the generated radical, and introducing a hydroxyl group, a carboxyl group, or the like by allowing the radical to react with oxygen (see, e.g., Patent Document 1). This realizes a carbon thin film with high hydrophilicity and excellent biocompatibility.

S Szunerits et al. in Electrochemistry Communications 8 (2006) 1185-1190 discloses an ammonia plasma treatment of a hydrogenated diamond surface in order to generate surface amino groups.

### SUMMARY OF THE INVENTION

### TECHNICAL PROBLEM

However, there arise the following problems in the conventional method of improving quality of the carbon thin film. In order to obtain cell chips such as cell microarrays and tissue microarrays, and materials having higher biocompatibility, promotion of adhesion of the cells and reduction in adsorption of the cells are desired to be controlled. In addition, it is preferable that cells allowing adhesion are not deactivated, and that cells not allowing adhesion can be selectively deactivated. A carbon thin film has little interaction with cells. Therefore, it is expected that cell chips and biocompatible materials, which are hardly deactivated and have high durability, can be obtained by using a base material coated with a carbon thin film.

However, since a carbon thin film has less interaction with various cells, it is almost impossible to immobilize the cells in most cases. As such, a conventional carbon thin film, which is hardly immobilized to cells of which adhesion is desired to be promoted, lacks properties as a biocompatible material. As a method of promoting or reducing adhesion of cells, modification of the surface of the carbon thin film can be considered. However, the functional groups, which can be introduced into a carbon thin film by conventional plasma irradiation, are a hydroxyl group, a carbonyl group, and a carboxyl group. Hydrophilicity of the carbon thin film can be improved by introducing the hydroxyl group, the carbonyl group, or the carboxyl group. However, the hydroxyl group, the carbonyl group, and the carboxyl group function as a barrier between the cells and the carbon thin film for cells allowed to adhere, and may reduce the amount of the cells. In particular, the cells often have negative charges, and the carbon thin film introduced with the carboxyl group also has a negative charge. The present inventors found that this causes electric repulsion between the cells and the carbon thin film, thereby reducing the amount of the cells to adhere.

Another possible solution is to increase surface potential (zeta potential) of the carbon thin film by allowing the carboxyl group to react and convert to other functional groups, and immobilizing other materials using the carboxyl group. However, such solid state reaction is difficult to control and increases manufacturing steps, thereby causing problems in practical use.

As such, there are difficulties in using conventional carbon thin films for surface coating of cell chips and medical materials. Furthermore, since DNA etc. have a negative charge, similar problems arise in DNA chips etc.

It is an objective of the present invention to solve the above-described problems and to realize a carbon thin film which has relatively high surface potential, and easily allows immobilization or reduced adsorption of biocomponents such as DNA and various cells such as blood platelets, endotheliums, and smooth muscle cells.

### SOLUTION TO THE PROBLEM

In order to achieve the objective, a carbon thin film of the present invention according to claim 1 is introduced with an amino group in the carbon framework.

Specifically, the carbon thin film according to the present invention includes a film body having carbon atoms bonded together; and an amino group bonded to the carbon atoms forming the film body.

As such, the carbon thin film of the present invention includes the amino group bonded to the carbon atoms forming the film body. Thus, the surface potential of the carbon thin film can be higher than that of a conventional carbon thin film not containing an amino group. This facilitates immobilization and reduction in adsorption of biomaterials such as various cells and DNA having negative charges. Therefore, different from the case where the surface of the DLC film is coated only with the carboxyl group; DNA chips, biochips, and high biocompatible materials, which sufficiently function, can be realized.

The carbon thin film of the present invention further includes a carboxyl group bonded to the carbon atoms forming the film body.

With this configuration, the amino group and the carboxyl group are balanced, thereby controlling the surface potential of the carbon thin film as required.

In the carbon thin film of the present invention, surface potential is -10 mV or more. Furthermore, a ratio of nitrogen to the total carbon is 0.05 or more.

In the carbon thin film of the present invention, the film body may contain silicon. In this case, the content of the silicon is preferably 5% or less.

The method of forming a carbon thin film of the present invention includes the steps of (a) forming on a surface of a base material, a film body having carbon atoms bonded together, and (b) introducing an amino group into the carbon atoms forming the film body by irradiating the film body with gas plasma containing ammonia.

As such, the method of forming the carbon thin film of the present invention includes the step of introducing the amino group into the carbon atoms forming the film body by irradiating the film body with the gas plasma containing ammonia. Thus, the amino group can be directly introduced into the carbon thin film in the single step. Therefore, the number of steps can be reduced and the amino group can be more efficiently introduced compared to the case where other functional groups are converted to an amino group. In the method of forming the carbon thin film, in the step (a), the film body having a carboxyl group may be formed.

In the method of forming the carbon thin film of the present invention, in the step (b), a carboxyl group is introduced together with the amino group.

In the method of forming the carbon thin film of the present invention, in the step (b), the film body may be irradiated with ammonia plasma after being irradiated with inert gas plasma, or may be irradiated with ammonia plasma after being irradiated with hydrocarbon plasma.

In the method of forming the carbon thin film of the present invention, in the step (b), surface potential is -10 mV or more. Furthermore, a ratio of nitrogen to the total carbon is 0.05 or more.

In the method of forming the carbon thin film of the present invention, the step (b) may include the step of irradiating the film body with oxygen plasma.

Furthermore, in the step (b), the film body may be irradiated with plasma of mixed gas of inert gas and ammonia, or with plasma of mixed gas of hydrocarbon and ammonia. In this case, the mixed gas may contain oxygen.

### ADVANTAGES OF THE INVENTION

According to the carbon thin film and the method of forming the film of the present invention, a carbon thin film can be realized, which has relatively high surface potential, and facilitates immobilization of biocomponents such as cells and DNA.

### BRIEF DESCRIPTION OF THE DRAWINGS

[FIG. 1] FIG. 1 is a schematic view of a plasma irradiation apparatus used in an embodiment of the present invention.
[FIG. 2] FIGS. 2(a)-(c) illustrate results of X-ray photoelectron spectroscopy of a carbon thin film obtained by irradiation with acetylene plasma and oxygen plasma in an embodiment of the present invention. FIG. 2(a) Illustrates a peak of C1s, FIG. 2(b) illustrates a peak of N1s, and FIG. 2 (c) illustrates a peak of O1s.
[FIG. 3] FIGS. 3(a)-(c) illustrate results of X-ray photoelectron spectroscopy of a carbon thin film obtained by irradiation with acetylene plasma and ammonia in an embodiment of the present invention. FIG. 3(a) illustrates a peak of C1s, FIG. 3(b) illustrates a peak of N1s, and FIG. 3(c) illustrates a peak of O1s.
[FIG. 4] FIGS. 4(a)-(c) illustrate results of X-ray photoelectron spectroscopy of a carbon thin film obtained by irradiation with argon plasma and ammonia plasma in an embodiment of the present invention. FIG. 4(a) illustrates a peak of C1s, FIG. 4(b) illustrates a peak of N1s, and FIG. 4(c) illustrates a peak of O1s.
[FIG. 5] FIG. 5 is a graph illustrating the content of an amino group and the content of a carboxyl group of a carbon thin film obtained in an embodiment of the present invention.
[FIG. 6] FIG. 6 is a graph illustrating the relationship between types of gas and surface potential of plasma of a carbon thin film obtained in an embodiment of the present invention.
[FIG. 7] FIG. 7 is a graph illustrating the relationship between the content of a carboxyl group and surface potential of a carbon thin film obtained in an embodiment of the present invention.
[FIG. 8] FIG. 8 is a graph illustrating the relationship between the silicon content and the introduction amount of the functional group of a carbon thin film obtained in an embodiment of the present invention.
[FIG. 9] FIG. 9 is a graph illustrating the relationship between the silicon content and the generation amount of silicon oxide after plasma irradiation of a carbon thin film obtained in an embodiment of the present invention.
[FIG. 10] FIGS. 10(a)-(c) illustrate results of X-ray photoelectron spectroscopy of a carbon thin film obtained by irradiation with ammonia plasma in an embodiment of the present invention. FIG. 10(a) illustrates a peak of C1s, FIG. 10(b) illustrates a peak of N1s, and FIG. 10(c) illustrates a peak of O1s.

### DESCRIPTION OF REFERENCE CHARACTERS

- 10: Chamber
- 11: Base Material
- 12A: Parallel Plate Electrode
- 12B: Parallel Plate Electrode
- 13: Mass Flow Controller
- 14: Matching Box
- 15: High-Frequency Power Supply

### DESCRIPTION OF EMBODIMENTS

The present inventors found that an amino group can be introduced into a carbon thin film such as a diamond-like carbon film formed on a surface of the base material by irradiating the carbon thin film with plasma. By introducing the amino group, surface potential (zeta potential) of the carbon thin film can be higher than that of a conventional film. Furthermore, the present inventors found that the introduction amount of the amino group can be changed, and a carboxyl group can be introduced with the amino group by changing the types of plasma used for irradiation. This freely changes surface potential of the carbon thin film.

Devices such as cell chips need to be capable of allowing immobilization of cells onto surfaces without deactivating cells. With respect to medical devices, only cells allowing adhesion can be preferably activated, and cells not allowing adhesion can be preferably deactivated. The surface potential of a device affects interaction between cells and the device, and thus, it is important to control the surface potential of a device to maintain immobilization of the cells to the device, reduction in adsorption, and activation of the cells. Therefore, a base material provided with a carbon thin film, which can be introduced with an amino group or an amino group and a carboxyl group to freely change the surface potential, provides excellent performance as a cell chip or a device made of, e.g., a biocompatible material may be used.

### Formation of Carbon thin film

First, formation of a carbon thin film will be described below. A base material forming a carbon thin film may be any material, as long as it can form a microwell, a DNA chip, a cell chip, a biocompatible material, and the like. The usage is not limited thereto. Any material may be used as long as it serves as a base material in various types of usage requiring smoothness, and control of durability and surface potential, for example, a resin material, a ceramics material, or a metal material.

Specifically, although not particularly limited thereto, for example, metal such as iron, nickel, chrome, copper, titanium, platinum, tungsten, or tantalum can be used as a base material. Also, an alloy of the materials including stainless steel such as SUS316L, a shape memory alloy such as a Ti-Ni alloy or a Cu-Al-Mn alloy, a Cu-Zn alloy, a Ni-Al alloy, a titanium alloy, a tantalum alloy, a platinum alloy, or a tungsten alloy can be used. Furthermore, the material may be non-bioactive ceramics or apatite having oxide, nitride, or carbide of aluminum, silicon, or zircon; or bioactive ceramics such as bioglass. Moreover, the material may be a polymer resin such as polymethylmethacrylate (PMMA), high-density polyethylene, or polyacetal; silicon polymer such as polydimethylsiloxane; fluorine polymer such as polytetrafluoroethylene.

The carbon thin film covering the surface of the base material is a film formed by Sp2 bonding and Sp3 bonding and represented by a diamond thin film. The film may include hydrogen, oxygen, silicon, fluorine, and the like.

The carbon thin film may be formed by a known method. The film can be formed on the surface of the base material by, for example, sputtering, DC magnetron sputtering, RF magnetron sputtering, chemical vapor deposition (CVD), plasma CVD, plasma ion implantation, superposed RF plasma ion implantation, ion plating, arc ion plating, ion beam deposition, or laser ablation. Although not limited thereto, the thickness of the film preferably ranges from 0.005 µm to 3 µm, more preferably, from 0.01 µm to 1 µm.

Furthermore, the carbon thin film may contain silicon (Si). When tetramethylsilane or the like which servers as a silicon source is supplied in addition to a carbon source when forming the carbon thin film, a carbon thin film containing Si can be formed. Similarly, fluorine or the like can be introduced.

While the carbon thin film can be directly formed on the base material, an interlayer may be formed between the base material and the carbon thin film to place the base material into more intimate contact with the carbon thin film. While, various materials are used for the interlayer according to the type of the base material, a known film such as an amorphous film made of silicon (Si) and carbon (C), titanium (Ti) and carbon (C), or chrome (Cr) and carbon (C). Although not limited thereto, the thickness of the film preferably ranges from 0.005 µm to 0.3 µm, more preferably, from 0.01 µm to 0.1 µm.

The interlayer can be formed by a know method. For example, sputtering, CVD, plasma CVD, spraying, ion plating, or arc ion plating may be used.

### Plasma Irradiation

Plasma irradiation of the carbon thin film may be performed by using a known plasma irradiation apparatus. Conditions for the plasma irradiation are not limited, but the irradiation is preferably performed without etching or with a small etching rate to reduce the damage to the carbon thin film.

The plasma irradiation may be performed in a single step, or two or more steps. In order to introduce the amino group, at least one irradiation stage may be the irradiation with ammonia plasma. In particular, if the film is irradiated with ammonia plasma after being irradiated with hydrocarbon plasma such as acetylene (C₂H₂) and benzene (C₆H₆), cleavage of carbon-carbon bonding and carbon-hydrogen, and introduction of the amino group proceed efficiently. Instead of hydrocarbon plasma, plasma of inert gas such as argon (Ar) may be used. Furthermore, by adding a step of irradiating the film with oxygen plasma, not only the amino group but also a carboxyl group can be introduced.

Furthermore, the film may be irradiated with plasma of mixed gas of ammonia and hydrocarbon or inert gas. Moreover, oxygen-mixed gas may be used.

### Composition Analysis

The composition of the obtained plasma-irradiated carbon thin film was assessed by X-ray Photoelectron Spectroscopy (XPS). For the measurement, photoelectron spectroscopic analyzer JPS-9010MC manufactured by JEOL Ltd. was used. A1 was used for an X-ray source, and an X ray was generated under the condition where the accelerating voltage is 12.5 kV, and the emission current is 17.5 mA.

### Analysis of Surface Potential

The surface potential of the obtained plasma-irradiated carbon thin film was measured as below. For the measurement, zeta potential/particle size measurement system ELS-Z manufactured by Otsuka Electronics was used. The obtained plasma-irradiated carbon thin film is placed in intimate contact with a cell for a plate sample, thereby injecting particles for monitoring into the cell. The particles for monitoring used here are those dispersed in sodium chloride (NaCl) solution of 10 mM, and manufactured by Otsuka Electronics. Electrophoresis of particles for monitoring is performed at each level in a cell depth direction to measure the apparent velocity distribution within the cell. The electrophoresis was performed under the condition where the average electric field is 17.33 V/cm, and the average current is 1.02 mA. The surface potential of the plasma-irradiated carbon thin film was obtained by analyzing the obtained apparent velocity distribution by the Mori-Okamoto equation. Note that the cell for the plate sample was used after being coated with polyacrylamide to reduce effects of charges on the cell surface.

Introduction of an amino group into the carbon thin film and the control of the surface potential of the carbon thin film will be described below in detail with the following embodiment.

### Embodiment

### Formation of Carbon thin film

In this embodiment, high-speed tool steel (JIS standard SKH51) of 12 mm per side and with a thickness of 5 mm is used as the base material.

The base material is set within a chamber in an ionized vapor deposition system and argon gas (Ar) is introduced into the chamber so that the pressure ranges from 10⁻¹Pa to 10⁻³ Pa (from 10⁻³ Torr to 10⁻⁵ Torr). Then, Ar ion is generated by discharge, and bombard cleaning is performed for 30 minutes to allow the generated Ar ion to collide with the surface of the base material. Then, tetramethylsilane (Si(CH₃)₄) is introduced for 3 minutes to form an interlayer in an amorphous state, which includes silicon (Si) and carbon (C) as main components, and has a thickness of 20 nm.

After forming the interlayer, C₆H₆ gas is introduced into the chamber under the gas pressure of 10⁻¹ Pa. C₆H₆ is ionized by performing discharge while continuously introducing C₆H₆ at the rate of 30 ml/min., thereby performing ionized evaporation vapor deposition for about two minutes to form the carbon thin film having a thickness of 30 nm on the surface of the base material.

When forming the DLC film, the target voltage was 1.5 kV, the target current was 50 mA, the filament voltage was 14 V, the filament current was 30 A, the anode voltage was 50 V, the anode current was 0.6 A, the reflector voltage was 50 V, the reflector current was 6 mA. The temperature of the base material during the formation was about 160°C.

Furthermore, by supplying tetramethylsilane as a silicon source at the same time when forming the carbon thin film, the carbon thin film is obtained, which has the Si content of 0 at.%, 3 at.%, 19 at.%, and 28.5 at.%. The Si content was calculated by an XPS analysis.

Note that the interlayer is provided to improve the adhesion between the base material and DLC film, and may be omitted where the adhesion between the base material and the DLC film can be sufficiently obtained.

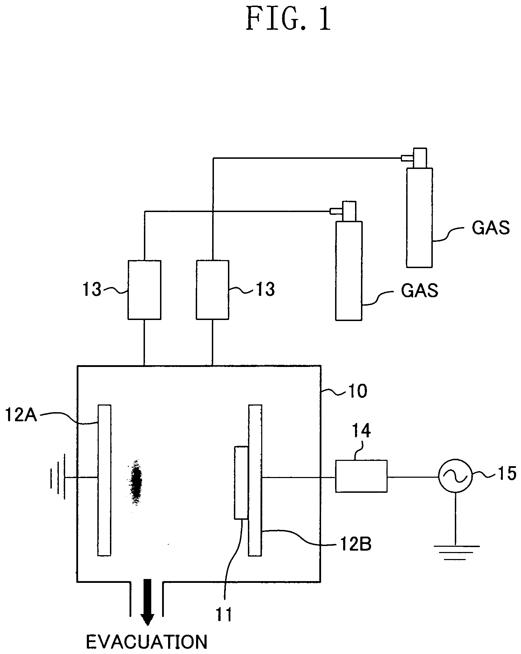

Then, the obtained carbon thin film is irradiated with plasma, thereby introducing a functional group. The plasma irradiation was performed with a plasma irradiation apparatus of a parallel plate type as shown in FIG. 1. After setting a base material 11 provided with a carbon thin film within a chamber 10 of the plasma irradiation apparatus, the air is evacuated until the pressure in the chamber 10 reaches 2 Pa. Next, gas is introduced into the chamber 10 at a predetermined flow rate and high-frequency power of 30 W is applied between parallel plate electrodes 12A and 12B, thereby generating plasma. The gas flow rate was controlled by the mass flow controller 13, and the pressure in the chamber during the plasma irradiation was 133 Pa. The high-frequency power was applied using a high-frequency power supply 15, which is coupled to an electrode via a matching box 14.

In this embodiment, five types of gas are used: argon (Ar), oxygen (O₂), acetylene (C₂H₂), ammonia (NH₃), and mixed gas (Ar/O₂) of Ar and O₂. The plasma irradiation was performed under eight conditions shown in Table 1. The irradiation time of plasma was 15 seconds per gas.

**[Table 1]**

| | Sample No. | | | | | | | |
|---|---|---|---|---|---|---|---|---|
| | 1 | 2 | 3 | 4 | 5 | 6 | 7 | 8 |
| Step 1 | O₂ | Ar | C₂H₂ | C₂H₂ | As/O₂ | Ar | Ar | C₂H₂ |
| Step 2 | None | None | None | O₂ | O₂ | O₂ | NH₃ | NH₃ |

### Result of Analysis

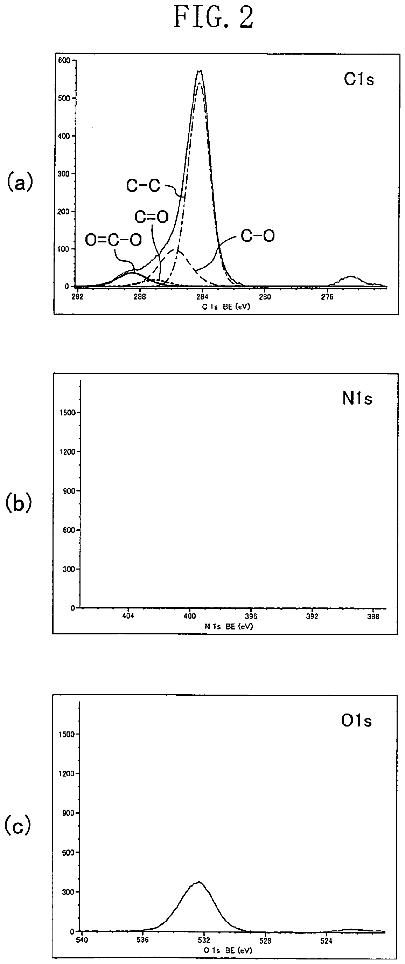

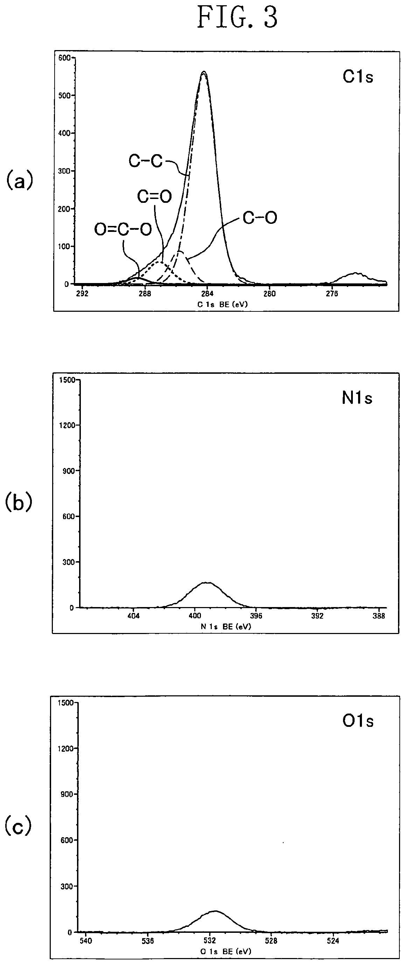

FIG. 2 illustrates results of XPS analysis where a film is irradiated with oxygen plasma after being irradiated with acetylene plasma, and FIG. 3 shows results of XPS analysis where the film is irradiated with ammonia plasma after being irradiated with acetylene plasma. Note that FIGS. 2 and 3 show the results of a carbon thin film having the Si content of 0%.

If the film is irradiated with acetylene plasma and oxygen plasma, the O1ₛ peak is significant as shown in FIG. 2(c). Furthermore, as shown in FIG. 2(a), the ratio of the carboxyl group (O=C-O) is high at the C1ₛ peak, showing that the carboxyl group is introduced. On the other hand, as shown in FIG. 3(c), the O1ₛ peak can also be seen, where the film is irradiated with acetylene plasma and ammonia plasma. However, the ratio of the carboxyl group at the peak of C1ₛ is extremely low as compared to the case where the film is irradiated with oxygen plasma. Therefore, it is apparent that the introduction amount of the carboxyl group is smaller in the combination of acetylene plasma and ammonia plasma, than in the combination of acetylene plasma and oxygen plasma.

Furthermore, as shown in FIG. 3(b), the N1s peak, which is not seen in the combination of acetylene plasma and oxygen plasma, is observed in the combination of acetylene plasma and ammonia plasma; and it is found that nitrogen (N) is introduced into the carbon framework of a carbon thin film. Furthermore, the N1s peaks at 398.9 eV. This value deviates from the binding energy (400±1 eV) of N1s of amine and amide, and it is apparent that the amino group is introduced into the carbon framework.

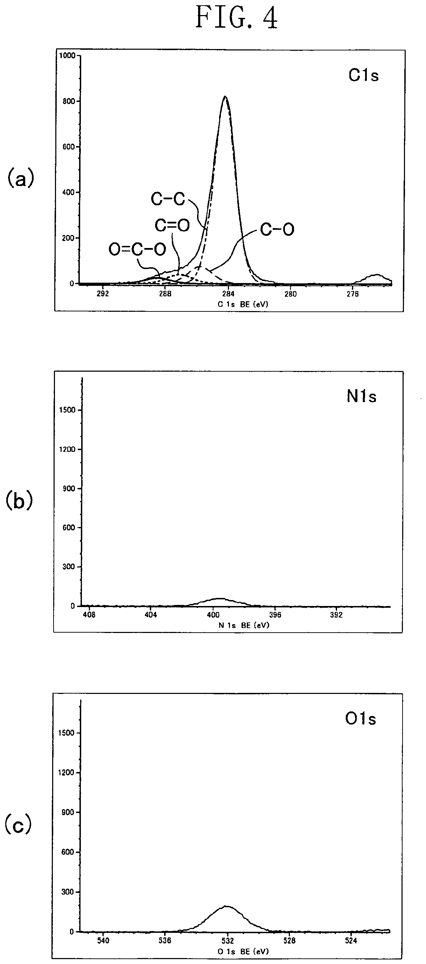

FIG. 4 illustrates results of XPS analysis where the film is irradiated with ammonia plasma after being irradiated with argon plasma. In this case, the N1s peak is also seen, and it is found that the amino group is introduced into the carbon framework. On the other hand, the ratio of the carboxyl group (O=C-O) at the C1s peak is higher than in the case where the film is irradiated with acetylene plasma and ammonia plasma. The introduction amount of the carboxyl group is larger than in irradiation with acetylene plasma.

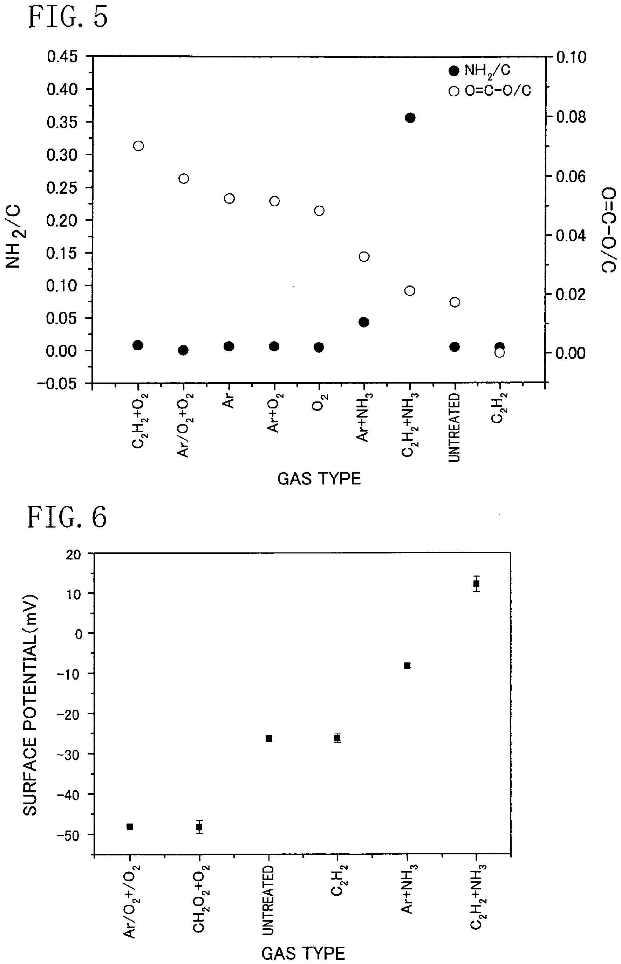

FIG. 5 illustrates the relationship between the types of gas used for plasma treatment, and the content of the carboxyl group (O=C-O) and the content of the amino group (NH₂). Note that, in FIG. 5, the content of the carboxyl group represents the ratio of the carboxyl group to the total carbon, and the content of the amino group represents the ratio of nitrogen to the total carbon. As shown in FIG. 5, the carbon thin film, which is not irradiated with plasma, also contains a carboxyl group. The content of the carboxyl group is about 0.02. This may be because; the carboxyl group is generated by reaction with oxygen in the atmosphere, when forming the carbon thin film. When the carbon thin film is irradiated only with acetylene plasma, the ratio of the carboxyl group decreases to almost zero. On the other hand, when the film is irradiated with plasma of gas types other than acetylene, or a mixture of acetylene plasma and plasma of another type of gas, the ratio of the carboxyl group increases as compared to an untreated carbon thin film. In particular, when the film is irradiated with oxygen plasma after being irradiated with acetylene plasma, the ratio of the carboxyl group is high at about 0.07. As such, by changing the conditions of the plasma irradiation, the content of the carboxyl group can be changed within the range from about 0 to about 0.07. On the other hand, when the film is irradiated with ammonia plasma, an amino group is generated, which is not contained in the untreated carbon thin film. The generation amount of the amino group is more in the case where the film is irradiated with ammonia plasma after being irradiated with acetylene plasma than in the case where the film is irradiated with ammonia plasma after being irradiated with argon plasma. Specifically, the content of the amino group is about 0.05, where the film is irradiated with ammonia plasma after being irradiated with argon gas plasma, and the content is over 0.35 where the film is irradiated with ammonia plasma after being irradiated with acetylene plasma.

As such, the reason is unclear why the amount of the functional group decreases, where the film is irradiated only with acetylene plasma; and why the amount of the functional group increases, where the film is continuously irradiated with acetylene plasma and oxygen plasma or ammonia plasma. However, numbers of the scaffoldings of the C-H bonding is generated on the surface of the carbon thin film, where the film is irradiated with acetylene plasma. Since the generated C-H bonding has lower bonding energy than the C-C bonding, the bonding can be easily cut by a radical or ion within oxygen plasma and ammonia plasma, thereby easily generating a dangling-bond. Thus, the radical of oxygen or ammonia can be extremely easily introduced into the surface of the carbon thin film. For this reason, numbers of carboxyl groups are generated where the film is continuously irradiated with oxygen plasma, and numbers of amino groups are generated where the film is irradiated with ammonia plasma. On the other hand, if plasma irradiation does not follow, the functional group is not generated since the rate at which acetylene plasma generates a C-C bonding and a C-H bonding is higher than the rate at which the functional group is generated by remaining oxygen.

As such, the introduction amounts of the amino group and the carboxyl group into the carbon thin film can be controlled by changing the gas type of plasma. The surface potential of the base material can be changed by controlling the introduction amounts of the amino group and the carboxyl group. FIG. 6 illustrates the relationship between the gas types of plasma used for irradiation, and the surface potential. As shown in FIG. 6, the surface potential is at a positive value over +10 mV, where the film is irradiated with ammonia plasma after being irradiated with acetylene plasma. The surface potential also increases to about -10 mV when the film is irradiated with ammonia plasma after being irradiated with argon plasma, in which the amino group is less introduced. When only the carboxyl group is introduced, the surface potential decreases as compared to the untreated film.

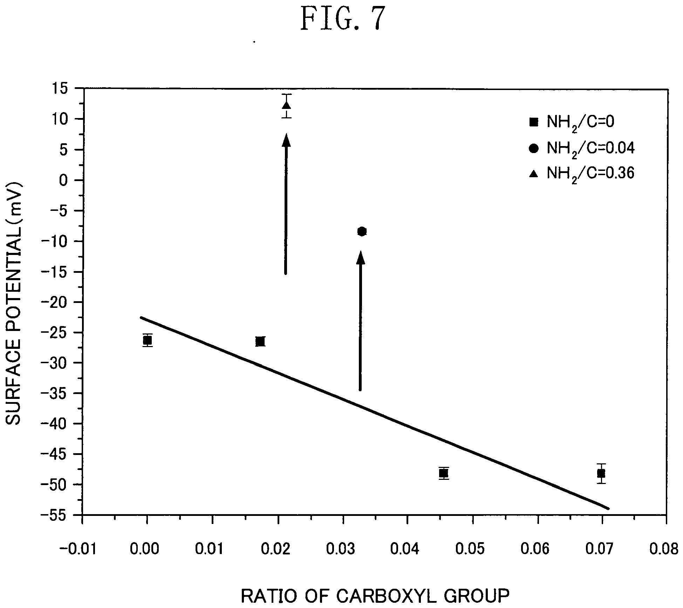

FIG. 7 illustrates the result of plotting values of the surface potential of the carbon thin film to the total carbon of the carboxyl group. When the amino group is not introduced, and the ratio of the carboxyl group increases; the value of the surface potential simply decreases. On the other hand, by introducing the amino group, the surface potential can be increased as compared to the case where only the carboxyl group is introduced. Specifically, the surface potential of the carbon film can be easily changed in the range from about -50 mV to about +15 mV. As such, by changing the rate of the introduction amount of the carboxyl group and the introduction amount of the amino group, the surface potential of the base material can be controlled.

In order to increase the ratio of the carboxyl group, the film may be irradiated with oxygen plasma in addition to, e.g., acetylene plasma and ammonia plasma, or argon plasma and ammonia plasma. On the other hand, in order to increase the ratio of the amino group, for example, irradiation time of ammonia plasma may be increased. In addition, the introduction amounts of the carboxyl group and the amino group can be controlled by controlling applied power, operational pressure, and degree of vacuum.

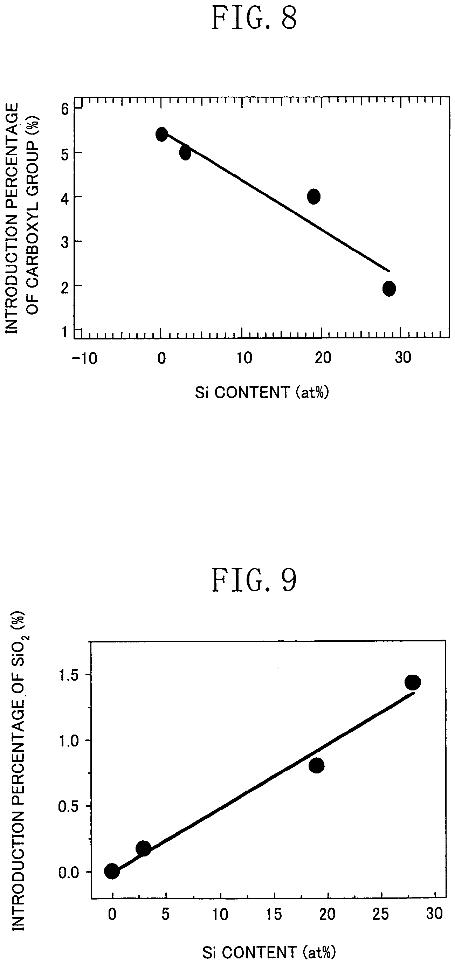

The carbon thin film introduced with the functional group preferably contains less than 5% of Si. FIG. 8 illustrates the introduction percentage of the carboxyl group where the carbon thin film having a different amount of Si is irradiated with oxygen plasma.

In FIG. 8, the vertical axis represents the difference between the percentage of the carboxyl group to the total carbon after the plasma irradiation, and the percentage of the carboxyl group to the total carbon before the plasma irradiation. As shown in FIG. 8, with an increase in the Si content, the introduction percentage of the carboxyl group decreases.

FIG. 9 illustrates the result of plotting the sample shown in FIG. 8, focusing on the introduction percentage of SiO₂. In FIG. 9, the vertical axis represents the ratio to the sum of the total Si amount and the total carbon amount in SiO₂. With an increase in the Si amount contained in the carbon thin film before plasma irradiation, the introduction ratio SiO₂ increases. In view of the foregoing, the oxygen radical in plasma, which reacts not with carbon but with Si, increases to reduce the introduction amount of the functional group; where the carbon thin film contains Si.

From the above result, in view of introducing the functional group such as the carboxyl group containing oxygen, the Si content of the carbon thin film is preferably as small as possible and less than 5%.

While in this embodiment, an example has been described where both of the carboxyl group and the amino group are introduced, a carbon thin film, which does not contain the carboxyl group in an untreated state, can be formed by changing the manufacturing conditions of the carbon thin film. In this case, a carbon thin film, which does not contain a carboxyl group and contains only an amino group, can be obtained by irradiating the film with ammonia plasma after irradiating with acetylene plasma. Furthermore, by controlling the conditions of the plasma irradiation, the carbon thin film containing only the amino group can be obtained, even when plasma of inert gas such as argon is used instead of acetylene plasma.

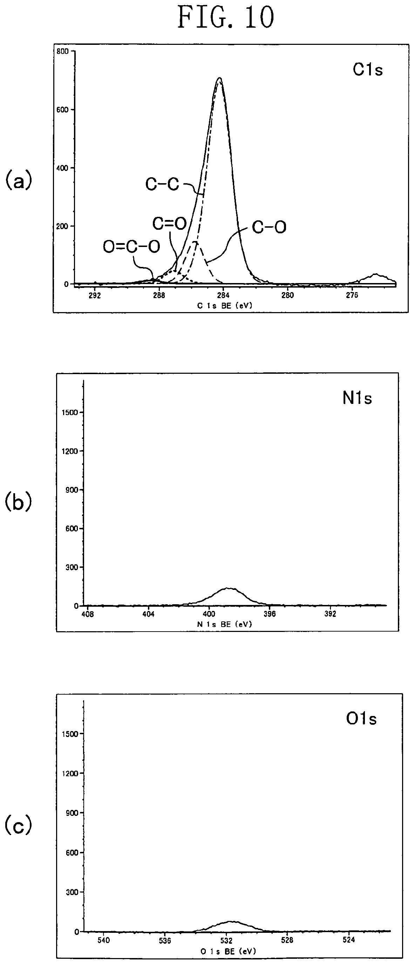

While an example has been described here where the second plasma treatment with ammonia plasma is performed after the first plasma treatment with, e.g., acetylene plasma and argon plasma, an amino group can be introduced by irradiating the film only with ammonia plasma. FIG. 10 illustrates an XPS spectrum where the film is irradiated only with ammonia plasma. As shown in FIG. 10, an N1s peak is detected, and it is found that the amino group is introduced. As described above, the amino group can be sufficiently introduced by irradiation only with ammonia plasma. Note that the amino group can be further effectively introduced by combining acetylene plasma, argon plasma, or the like with ammonia plasma.

### INDUSTRIAL APPLICABILITY

The carbon thin film and the method of forming the film according to the present invention realize a carbon thin film containing an amino group and having relatively high surface potential. Thus, the film and the method are useful particularly as a carbon thin film, which can be a base material such as a biochip and a DNA chip bonding biocomponents such as cells and DNA as well as a biocompatible material requiring immobilization of various types of cell and reduction in adsorption, and a method of forming the film.

## Claims

1. A carbon thin film, comprising:
(i) a film body having carbon atoms bonded together;
(ii) an amino group bonded to the carbon atoms forming the film body; and
(iii) a carboxyl group bonded to the carbon atoms forming the film body;
**characterized in that**
the ratio of nitrogen to the total carbon is 0.05 or more; and
the surface potential is -10 mV or more.

2. The carbon thin film of claim 1, wherein the film body contains silicon.

3. The carbon thin film of claim 2, wherein the content of the silicon is 5% or less.

4. The carbon thin film of any of claims 1 to 3, wherein the ratio of carboxyl to carbon is 0.07 or less; and wherein the ratio of the nitrogen is higher than the ratio of carboxyl.

5. A method of forming a carbon thin film according to any one of claims 1 to 4 comprising the steps of:
(a) forming the film body on a surface of a base material; and
(b) introducing an amino group into the carbon atoms forming the film body by irradiating the film body with gas plasma containing ammonia.

6. The method of forming the carbon thin film of claim 5, wherein in the step (a), the film body having a carboxyl group is formed.

7. The method of forming the carbon thin film of claim 5, wherein in the step (b), a carboxyl group is introduced together with the amino group.

8. The method of forming the carbon thin film of claim 5, wherein in the step (b), the film body is irradiated with ammonia plasma after being irradiated with inert gas plasma.

9. The method of forming the carbon thin film of claim 5, wherein in the step (b), the film body is irradiated with ammonia plasma after being irradiated with hydrocarbon plasma.

10. The method of forming the carbon thin film of claim 5, wherein the step (b) includes the step of irradiating the film with oxygen plasma.

11. The method of forming the carbon thin film of claim 5, wherein in the step (b), the film body is irradiated with plasma of mixed gas of inert gas and ammonia.

12. The method of forming the carbon thin film of claim 5, wherein in the step (b), the film body is irradiated with plasma of mixed gas of hydrocarbon and ammonia.

13. The method of forming the carbon thin film of claim 12, wherein the mixed gas contains oxygen.

## Patentansprüche

1. Kohlenstoff-Dünnfilm, umfassend:
(i) einen Filmkörper mit aneinander gebundenen Kohlenstoffatomen;
(ii) eine Aminogruppe, die an die den Filmkörper bildenden Kohlenstoffatome gebunden ist; und
(iii) eine Carboxylgruppe, die an die den Filmkörper bildenden Kohlenstoffatome gebunden ist;
**dadurch gekennzeichnet, dass**
das Verhältnis von Stickstoff zu dem Gesamtkohlenstoff 0,05 oder mehr beträgt; und das Oberflächenpotential -10 mV oder mehr beträgt.

2. Kohlenstoff-Dünnfilm nach Anspruch 1, wobei der Filmkörper Silicium enthält.

3. Kohlenstoff-Dünnfilm nach Anspruch 2, wobei der Anteil an Silicium 5% oder weniger beträgt.

4. Kohlenstoff-Dünnfilm nach einem der Ansprüche 1 bis 3, wobei das Verhältnis von Carboxyl zu Kohlenstoff 0,07 oder weniger beträgt; und wobei das Verhältnis des Stickstoffs höher als das Verhältnis von Carboxyl ist.

5. Verfahren zum Ausbilden eines Kohlenstoff-Dünnfilms nach einem der Ansprüche 1 bis 4, welches die folgenden Schritte umfasst:
(a) Bilden des Filmkörpers auf einer Oberfläche eines Grundmaterials; und
(b) Einbringen einer Aminogruppe in die Kohlenstoffatome, welche den Filmkörper bilden, durch Bestrahlen des Filmköpers mit gasplasmahaltigem Ammoniak.

6. Verfahren zum Ausbilden des Kohlenstoff-Dünnfilms nach Anspruch 5, wobei in Schritt (a) der eine Carboxylgruppe aufweisende Filmkörper ausgebildet wird.

7. Verfahren zum Ausbilden des Kohlenstoff-Dünnfilms nach Anspruch 5, wobei in Schritt (b) zusammen mit der Aminogruppe eine Carboxylgruppe eingebracht wird.

8. Verfahren zum Ausbilden des Kohlenstoff-Dünnfilms nach Anspruch 5, wobei in Schritt (b) der Filmkörper nach Bestrahlen mit Inertgasplasma mit Ammoniakplasma bestrahlt wird.

9. Verfahren zum Ausbilden des Kohlenstoff-Dünnfilms nach Anspruch 5, wobei in Schritt (b) der Filmkörper nach Bestrahlen mit Kohlenwasserstoffplasma mit Ammoniakplasma bestrahlt wird.

10. Verfahren zum Ausbilden des Kohlenstoff-Dünnfilms nach Anspruch 5, wobei der Schritt (b) den Schritt des Bestrahlens des Films mit Sauerstoffplasma umfasst.

11. Verfahren zum Ausbilden des Kohlenstoff-Dünnfilms nach Anspruch 5, wobei in Schritt (b) der Filmkörper mit Plasma von Mischgas aus Inertgas und Ammoniak bestrahlt wird.

12. Verfahren zum Ausbilden des Kohlenstoff-Dünnfilms nach Anspruch 5, wobei in Schritt (b) der Filmkörper mit Plasma von Mischgas aus Kohlenwasserstoff und Ammoniak bestrahlt wird.

13. Verfahren zum Ausbilden des Kohlenstoff-Dünnfilms nach Anspruch 12, wobei das Mischgas Sauerstoff enthält.

## Revendications

1. Film mince de carbone, comprenant :
(i) un corps de film ayant des atomes de carbone liés ensemble ;
(ii) un groupe amino lié aux atomes de carbone formant le corps de film ; et
(iii) un groupe carboxyle lié aux atomes de carbone formant le corps de film ;
**caractérisé en ce que**
le rapport de l'azote au carbone total est de 0,05 ou plus ; et
le potentiel de surface est de -10 mV ou plus.

2. Film mince de carbone selon la revendication 1, dans lequel le corps de film contient du silicium.

3. Film mince de carbone selon la revendication 2, dans lequel la teneur en silicium est de 5 % ou moins.

4. Film mince de carbone selon l'une quelconque des revendications 1 à 3, dans lequel le rapport du carboxyle au carbone est de 0,07 ou moins ; et dans lequel le rapport de l'azote est supérieur au rapport de carboxyle.

5. Procédé de formation d'un film mince de carbone selon l'une quelconque des revendications 1 à 4 comprenant les étapes consistant à :
(a) former le corps de film sur une surface d'un matériau de base ; et
(b) introduire un groupe amino dans les atomes de carbone formant le corps de film en irradiant le corps de film avec un plasma gazeux contenant de l'ammoniac.

6. Procédé de formation du film mince de carbone selon la revendication 5, dans lequel à l'étape (a), le corps de film ayant un groupe carboxyle est formé.

7. Procédé de formation du film mince de carbone selon la revendication 5, dans lequel à l'étape (b), un groupe carboxyle est introduit conjointement au groupe amino.

8. Procédé de formation du film mince de carbone selon la revendication 5, dans lequel à l'étape (b), le corps de film est irradié avec un plasma d'ammoniac après avoir été irradié avec un plasma de gaz inerte.

9. Procédé de formation du film mince de carbone selon la revendication 5, dans lequel à l'étape (b), le corps de film est irradié avec un plasma d'ammoniac après avoir été irradié avec un plasma d'hydrocarbure.

10. Procédé de formation du film mince de carbone selon la revendication 5, dans lequel l'étape (b) inclut l'étape consistant irradier le film avec un plasma d'oxygène.

11. Procédé de formation du film mince de carbone selon la revendication 5, dans lequel à l'étape (b), le corps de film est irradié avec un plasma d'un gaz mixte de gaz inerte et d'ammoniac.

12. Procédé de formation du film mince de carbone selon la revendication 5, dans lequel à l'étape (b), le corps de film est irradié avec un plasma d'un gaz mixte d'hydrocarbure et d'ammoniac.

13. Procédé de formation du film mince de carbone selon la revendication 12, dans lequel le gaz mixte contient de l'oxygène.
